Europäisches Patentamt

European Patent Office

Office européen des brevets

(19)

(11) Veröffentlichungsnummer : **0 130 549**
**B1**

(12) **EUROPÄISCHE PATENTSCHRIFT**

(45) Veröffentlichungstag der Patentschrift :
04.03.87

(21) Anmeldenummer : 84107387.7

(22) Anmeldetag : 27.06.84

(51) Int. Cl.⁴ : **C 07 C101/74, C 07 C 99/00,
A 61 K 31/60**

(54) **5-Aminosalicylsäure-O-sulfate von physiologisch unbedenklichen Basen, Verfahren zu ihrer Herstellung und diese enthaltende Arzneimittel.**

(30) Priorität : 01.07.83 DE 3323702

(43) Veröffentlichungstag der Anmeldung :
09.01.85 Patentblatt 85/02

(45) Bekanntmachung des Hinweises auf die Patenterteilung : 04.03.87 Patentblatt 87/10

(84) Benannte Vertragsstaaten :
AT BE CH DE FR GB IT LI LU NL SE

(56) Entgegenhaltungen :
DE-A- 2 156 556
JOURNAL OF THE AMERICAN CHEMICAL SOCIETY, Band 51, November 1929; K.H. ENGEL "Cleavage of azo dyes by means of sulfites. The cleavage of 4-hydroxy-azobenzene-5-carboxylic a and of 3-methyl-4-hydroxy-azobenzene-5-carboxy acid.", Seiten 3483-3489
CHEMICAL ABSTRACTS, Band 79, Nr. 9, 3. September 1973, Columbus, Ohio, USA; O. JACOBI "Nucleotide-amino acid adducts", Seite 402, Spalte 2, Zusammenfassung Nr. 53744h, in Verbindung mit Formel Register Seite 269F, Zeile 85
CHEMICAL ABSTRACTS, Band 46, Nr. 5, 10. März 1952, Columbus, Ohio, USA; J. PARROD et al. "The sulfuric acid ester of 4-aminosalicylic acid", Spalte 2013

(73) Patentinhaber : Henning Berlin GmbH Chemie und Pharmawerk
Komturstrasse 19-20
D-1000 Berlin 42 (DE)

(72) Erfinder : Rokos, Hartmut, Dr.
Am Fischtal 27b
D-1000 Berlin 37 (DE)
Erfinder : Konczak, Heinz
Rathausstrasse 23
D-1000 Berlin 42 (DE)
Erfinder : Forth, Wolfgang, Prof. Dr. med.
Volpinistrasse 54
D-8000 München 19 (DE)

(74) Vertreter : Werner, Hans-Karsten, Dr. et al
Deichmannhaus am Hauptbahnhof
D-5000 Köln 1 (DE)

## Beschreibung

Gegenstand der vorliegenden Erfindung sind 5-Aminosalicylsäure-O-sulfate von physiologisch unbedenklichen Basen, Verfahren zu ihrer Herstellung und diese enthaltene Arzneimittel, die zur Behandlung von Colitis ulcerosa, Enteritis regionalis Crohn (Morbus Crohn), chronischer unspezifischer Colitis und Divertikulitis geeignet sind.

Das Standardpräparat zur Behandlung dieser Krankheiten ist bisher Salazosulfapyridin. Diese Verbindung wird meist oral verabreicht und wahrscheinlich im Dickdarm bakteriell in die beiden Metaboliten 5-Aminosalicylsäure und Sulfapyridin gespalten. Die wirksame Komponente scheint der Metabolit 5-Aminosalicylsäure zu sein, während die häufig beobachteten Nebenwirkungen auf den Metaboliten Sulfapyridin zurückzuführen sind ; vgl. KHAN, et al. Lancet 2, 892 (1977). Da 5-Aminosalicylsäure instabil ist und auf oralem Weg praktisch nicht bis in den Dickdarm transportiert werden kann, wurde bereits von KHAN, loc. cit. die Möglichkeit erwogen, eine neue Substanz zu synthetisieren, die diese Nachteile des bekannten Salazosulfapyridins nicht aufweist.

Aus der DE-A-30 27 013 der Anmelderin ist ein Mittel zur Behandlung von Colitis ulcerosa, Enteritis regionalis Crohn (Morbus Crohn), chronischer unspezifischer Colitis und Divertikulitis bekannt, welches Salicylazobenzoesäure enthält. Diese Mittel werden zur Zeit mit Erfolg klinisch geprüft. Die Salicylazobenzoesäure enthält aber eine Azogruppierung, die als toxikologisch bedenklich gilt und daher nach Möglichkeit in Arzneimitteln vermieden werden sollte.

Die Erfindung hat sich somit die Aufgabe gestellt, andere Derivate der 5-Aminosalicylsäure zu entwickeln, welche die Nachteile des Salazosulfapyridins vermeiden und zumindest in vergleichbarer Weise wie die Salicylazobenzoesäure therapeutisch eingesetzt werden können.

Es wurde jetzt gefunden, daß 5-Aminosalicylsäure-O-sulfate von physiologisch unbedenklichen Basen hierzu ausgezeichnet geeignet sind und darüberhinaus den Vorteil aufweisen, länger und gleichmäßiger die gewünschte 5-Aminosalicylsäure im Dickdarm freizusetzen.

5-Aminosalicylsäure-O-sulfate von physiologisch unbedenklichen Basen sind bisher in der Literatur nicht beschrieben. Aus dem Register von Chemical Abstracts geht zwar hervor, daß « Benzoic acid, -5-amino-2-sulfoxy » bereits beschrieben sei, in Chemical Abstracts 79, 53744 (1973) wird jedoch nur die DE-A-21 56 556 referiert, in der eindeutig nur das 5-Amino-3-sulfosalicylat von Nucleotidaminosäure-Produkten beschrieben wird. Die 5-Aminosulfosalicylsäure ist eine Sulfonsäure, die durch Sulfonierung von Aminosalicylsäure entsteht. Die 5-Aminosalicylsäure-O-sulfate der vorliegenden Erfindung hingegen sind keine Sulfonsäuren sondern Schwefelsäureester der phenolischen OH-Gruppe der Aminosalicylsäure. Diese Schwefelsäureester sind durch direkte Synthese bisher nicht herstellbar gewesen.

In Chem. Abstracts 46 (1952), 2013a ist ferner ein 4-Aminosalicylsäure-O-schwefelsäureester erwähnt. Allerdings werden dort keinerlei pharmakologische Wirkungen angegeben.

Erfindungsgemäß können die 5-Aminosalicylsäure-O-sulfate erstmals dadurch hergestellt werden, daß man 5-Nitrosalicylsäure-alkylester mit Carbodiimid und Schwefelsäure verestert, die Akylestergruppe alkalisch verseift und die so erhaltenen 5-Nitrosalicylsäure-O-sulfate reduziert.

Als Alkylester kommen prinzipiell alle niederen Alkylester in Frage. Vorzugsweise wird der aus der Literatur bekannte Ethylester eingesetzt ; vgl. P. Thieme, J. für Prakt. Chem. 43, 453 (1891). In gleicher Weise könnten aber auch der Methylester, Propylester oder Butylester eingesetzt werden.

Die Veresterung der phenolischen OH-Gruppe erfolgt erfindungsgemäß durch Umsetzung mit Carbodiimiden und Schwefelsäure. Als Carbodiimide können alle für die Veresterung geeigneten üblichen Carbodiimide eingesetzt werden. Vorzugsweise wird Dicyclohexylcarbodiimid verwendet. In den nächsten Stufen werden in beliebiger Reihenfolge die Akylestergruppe alkalisch verseift und die 5-Nitrogruppe zur 5-Aminogruppe reduziert. Die Verseifung der Alkylestergruppe muß alkalisch erfolgen, da die Schwefelsäureester der Phenolgruppe gegen Säuren empfindlich sind. Die Empfindlichkeit gegen Säuren ist so stark, daß bereits die freie 5-Aminosalicylsäure-O-schwefelsäure sich bereits nach kurzer Zeit selbst zersetzt. Die Reduktion der 5-Nitrogruppe zur 5-Aminogruppe erfolgt in an sich bekannter Weise, besonders einfach durch katalytische Hydrierung bei Normaldruck oder erhöhtem Druck.

Je nachdem mit welcher Base die alkalische Verseifung der Estergruppe erfolgt, fällt bereits das entsprechende Salz des 5-Aminosalicylsäure-O-schwefelsäureesters an. Wegen seiner Kristallisationsfähigkeit und damit leichten Möglichkeit der Reinigung wird vorzugsweise Kalilauge als Base verwendet, zumal diese zu den physiologisch unbedenklichen Basen zählt. Es sind jedoch auch andere Basen, z. B. Natriumhydroxid, geeignet.

Die Überführung der Dikaliumsalze in andere Salze physiologisch unbedenklicher Basen erfolgt in an sich bekannter Weise, beispielsweise mit Hilfe von entsprechend aufgeladenen Ionenaustauschern.

Aus einer Arbeit von Engel über die Spaltung von Azofarbstoffen mit Hilfe von Sulfiten (J. Am. Chem. Soc. 51, S. 3483 (1929)) geht hervor, daß man vermutet hat, unter anderem auch den 5-Aminosalicylsäure-O-schwefelsäureester in den Händen gehabt zu haben. Hierbei hat es sich um eine offensichtliche Fehlinterpretation experimenteller Ergebnisse gehandelt, denn die Eigenschaften des damals gefundenen Produktes stimmen in keiner Weise mit denen des wirklichen 5-Aminosalicylsäure-O-schwefelsäureesters bzw. der erfindungsgemäßen 5-Aminosalicylsäure-O-sulfate von physiologisch unbedenklichen Basen überein. Unter anderem war die dort beschriebene Substanz gegen starke Säuren beständig und zersetzte sich erst nach längerem Kochen in 5 molarer Salzsäure in geringem Maße. Außerdem sprechen die Experimente zur direkten Herstellung eines 5-Aminosalicylsäure-O-schwefelsäureesters dagegen, daß man damals überhaupt diese Substanz in Händen gehabt haben könnte.

Die erfindungsgemäßen 5-Aminosalicylsäure-O-sulfate von physiologisch unbedenklichen Basen weisen die überraschende Eigenschaft auf, im Dünndarm praktisch nicht gespalten zu werden, jedoch im Dickdarm wahrscheinlich durch die dort vorhandenen Bakterien und Enzyme langsam und gleichmäßig in die gewünschte 5-Aminosalicylsäure und die physiologisch unbedenklichen Sulfate gespalten zu werden. Die erfindungsgemäßen Substanzen gestatten somit in idealer Weise den Transport der 5-Aminosalicylsäure an den Ort ihrer gewünschten Wirksamkeit.

Die Erfindung betrifft somit weiterhin Arzneimittel enthaltend 5-Aminosalicylsäure-O-sulfate von physiologisch unbedenklichen Basen. Es war sicherlich nicht vorhersehbar, daß die relativ niedermolekularen Salze von Schwefelsäureestern im Dünndarm praktisch nicht resorbiert werden, jedoch im Dickdarm in der gewünschten Weise gespalten werden und damit zur Verfügung stehen. Die erfindungsgemäßen Arzneimittel können in Form von Tabletten, Dragees aber auch als Klysmen eingesetzt werden. Da die Spaltung sowieso erst im Dickdarm oder Enddarm erfolgen soll, sind die erfindungsgemäßen Tabletten und Dragees vorzugsweise im Dünndarm löslich. Hierzu wählt man z. B. eine Rezeptur, in der die Stoffe nur im alkalischen Milieu des Dünndarms aufgelöst werden. Einfacher erreicht man dieses Ziel jedoch durch Überziehen der Tabletten oder Dragees mit einem dünndarmlöslichen Film, wie er beispielsweise mit den Eudragitlacken der Firma Röhm herstellbar ist.

Bei der Herstellung der erfindungsgemäßen Arzneimittel können alle üblichen Hilfsstoffe zur Herstellung von Tabletten und Dragees zur Anwendung kommen. Zu diesen üblichen Hilfsstoffen zählen Talkum, Stärke, Cellulose, Polyvinylpyrrolidon, Polyvinylpolypyrrolidon, Magnesiumstearat. Für die Herstellung von dünndarmlöslichen Rezepturen eignen sich insbesondere Polycarbonsäuren und andere Polymere, die nur im Alkalischen gespalten oder gelöst werden. Auch die üblichen dünndarmlöslichen Filmüberzüge bestehen meist aus derartigen sauren Polymeren, die erst im alkalischen Milieu des Dünndarms gelöst oder gespalten werden.

Die physiologisch unbedenkliche Base spielt bezüglich der Wirksamkeit und Resorption keine entscheidende Rolle. Es können daher praktisch anstelle des bereits oben erwähnten Kaliums auch Natrium, Calcium, Magnesium, Ammoniak und physiologisch akzeptable Amine als Basen eingesetzt werden.

Die erfindungsgemäßen Tabletten oder Dragees enthalten im allgemeinen 0,3 bis 1 g 5-Aminosalicylsäure-O-sulfate. Für die Behandlung akuter entzündlicher Prozesse werden Erwachsenen täglich 1 bis 6 g des Wirkstoffes oral verabreicht. Zur Rezidivprophylaxe in der Dauertherapie reichen 1 bis 4 g in mehreren Einzeldosen über den Tag verteilt. Da die bisher beobachteten Nebenwirkungen bei der Therapie mit Salazosulfapyridin bei den erfindungsgemäßen Mitteln nicht beobachtet werden, kann erfindungsgemäß auch höher als bisher dosiert werden.

Die Therapie erfolgt im allgemeinen oral, wobei bevorzugt dünndarmlösliche Tabletten oder Filmdragees zur Anwendung kommen. Unter besonderen Umständen, insbesondere wenn die Verweilzeit im Darm stark reduziert ist, können auch magenlösliche Tabletten oder Dragees zur Anwendung kommen.

Insbesondere bei stationärer Behandlung kommt anstelle der oralen Therapie auch die Einführung von Klysmen in Frage, wobei ein- oder mehrmals pro Tag bis 100 ml Klysma eingeführt werden können. Ein derartiges Klysma sollte 1 bis 6, vorzugsweise 3 g 5-Aminosalicylsäure-O-sulfate in 100 ml Lösung enthalten. Vorbereitete Klysmen sollten somit 10 bis 60, vorzugsweise 30 g Wirkstoff/l Klysma enthalten.

Aus den folgenden Beispielen geht die Herstellung und Wirksamkeit der erfindungsgemäßen 5-Aminosalicylsäure-O-sulfate von physiologisch unbedenklichen Basen hervor.

Beispiel 1

Im Eisbad wird zu einer Lösung von 74,26 g N,N'-Dicyclohexylcarbodiimid (DCC) in 80 ml Dimethylformamid (DMF) eine kalte Lösung von 10,14 g 5-Nitrosalicylsäureethylester (P. Thieme, J. für Prakt. Chem. 43, 453 (1891)) in 60 ml DMF eingerührt. Danach werden 3,8 ml konzentrierte Schwefelsäure in 60 ml DMF zugegeben. Die Suspension wird 1 h im Eisbad, anschließend 22 h bei Raumtemperatur gerührt.

Nach Abkühlen im Eisbad wird eine kalte Lösung von 6,75 g $K_2CO_3$ (wasserfrei) in 50 ml Wasser zugegeben. Diese Mischung wird in 2,4 l Eiswasser eingerührt. Nach 75 min. im Eisbad wird vom ausgefallenen Dicyclohexylharnstoff abgetrennt. Der Filterrückstand wird 2 x mit je 150 ml Wasser nachgewaschen. Das gelbe Filtrat wird zum Sirup eingeengt ; dieser kristallisiert nach Zugabe von 1 500 ml Diethylether und 60 ml Ethanol. Nach Durchkristallisation wird abgesaugt, mehrmals mit Diethylether gewaschen und im Exsikkator getrocknet.

Ausbeute : 16,7 g 5-Nitrosalicylsäureethylester-O-sulfat (Kaliumsalz)

Dünnschichtchromatographie : Kieselgel ; iso-Propanol/Chloroform/Methanol/Wasser (37/37/19/7, V/V) $R_f$ = 0,74

16,7 g 5-Nitrosalicylsäureethylester-O-sulfat (Kaliumsalz) werden in 790 ml verdünnter Kalilauge (4,4 g KOH) gelöst und zur Verseifung bei Raumtemperatur belassen.

3

Anschließend wird unter Rühren verdünnte Essigsäure langsam bis pH 8 eingerührt. Nach Zugabe von 820 ml Wasser und 3,16 g Pd-Katalysator (10 % auf BaSo₄, Degussa Typ E 50 N) wird ein kräftiger Wasserstoffstrom durchgeleitet (zur Beschleunigung der Reduktion kann im Druckgefäß gearbeitet werden).

Nach völliger Reduktion (Dünnschichtchromatographie, s. o.) wird vom Katalysator abgesaugt und das Filtrat auf 90 ml eingeengt. Das Konzentrat wird unter Rühren mit 390 ml wasserfreiem Ethanol versetzt. Nach Durchkristallisation wird abgesaugt, gewaschen und im Exsikkator getrocknet.

Ausbeute : 9,9 g 5-Aminosalicylsäure-O-sulfat (Dikaliumsalz).

Dünnschichtchromatographie : Kieselgel ; iso-Propanol/Chloroform/Methanol/Wasser (37/37/19/7, V/V/) R_f = 0.0

Die Spaltung des Schwefelsäureesters mit 2 N Salzsäure bei 110 °C oder mit Arylsulfatase (EC 3.1.6.1.) (Boehringer Mannheim) ergab 5-Aminosalicylsäure (Dünnschichtchromatographie im obigen System, R_f = 0.38).

Zur Analyse wurde aus wäßriger Lösung mit Ethanol nochmals umgefällt. Farblose Kristalle, Schmelzpunkt > 280 °C.

Analyse : $C_7H_5K_2NO_6S \times H_2O$ (327.4)

berechnet : C 25,7  H 2,2  K 23,9  N 4,3  S 9,8 %
gefunden : C 25,6  H 2,1  K 23,8  N 4,2  S 9,8 %

### Beispiel 2

Resorption von 5-Aminosalicylsäure-O-sulfatdikaliumsalz (ASA-S) mit 5-Aminosalicylsäure (ASA) und Salicylazobenzoesäure (HB-313).

Getestet wurden jeweils die Resorption am Dünndarm der Ratte (je 2 bis 5 Tiere). ASA und ASA-S wurden mittels HPLC und FLuoreszensdetektor bestimmt :

Säule RP-18 (5 μm), 120 × 4,6 mm
Fließmittel Acetonitril/0.2 M Phosphat, pH 7.5/1.25 mM. Cetyltrimethylammoniumbromid (30/5/65 ; V, V, V)
Flow 1 ml/min
Detektor Fluoreszens, 315/500 nm

ASA

| | | |
|---|---|---|
| Ausgangswert | 5.41 μg/ml | (100%) |
| 20 min | 4.87 ± 0.39 | 90.0 |
| 40 min | 4.26 ± 0.76 | 78.7 |
| 60 min | 3.12 ± 0.39 | 57.7 |

ASA-S

| | | |
|---|---|---|
| Ausgangswert | 10.93 μg/ml | (100%) |
| 20 min | 10.65 ± 1.60 | 97.4 |
| 40 min | 12.13 ± 1.05 | 110.9 |
| 60 min | 10.37 ± 0.91 | 94.9 |

2 ml der Dioxanlösung wurden mit Isoamylacetat extrahiert, mit Natronlauge rückextrahiert und bei 448 nm gemessen :

HB-313

| | | |
|---|---|---|
| Ausgangswert | 8.72 μg/ml | (100%) |
| 20 min | 8.75 ± 1.21 | 100.3 |
| 40 min | 8.58 ± 0.95 | 98.4 |
| 60 min | 9.00 ± 0.86 | 103.2 |

### Beispiel 3

4

# 0 130 549

Resorption und Spaltung an einem freiwilligen Probanden

| Wirkstoff | ASA-S | HB-313 |
|---|---|---|
| Einmaldosis (g) | 2.74 | 3.00 |
| (entsprechend g ASA) | (1.36) | (1.60) |

Ac-ASA Serumspiegel (µg/ml)

| | | |
|---|---|---|
| 6 h | 1.10 | 0.12 |
| 8 h | 0.97 | 0.46 |
| 12 h | 0.52 | 0.98 |
| 24 h | - | 0.65 |
| 25 h | 0.50 | - |

nach Einnahme

Ac-ASA kumulierte Urinausscheidung (% der Dosis)

| | | |
|---|---|---|
| 24 h | 4.91 | 9.17 |
| 48 h | 8.16 | 10.02 |

nach Einnahme

ASA 5-Aminosalicylsäure
ASA-S 5-Aminosalicylsäure-O-sulfat
Ac-ASA N-Acetyl-5-aminosalicylsäure
HB-313 Salicylazobenzoesäure

Die obigen Ergebnisse zeigen, daß ASA-S in gleicher Weise wie HB-313 im Dünndarm praktisch nicht resorbiert wird, jedoch im Dickdarm dann gleichmäßig und über einen längeren Zeitraum als HB-313 zu ASA freigesetzt, resorbiert und über den Urin ausgeschieden wird. Bei dem Probanden wurden keinerlei Nebenwirkungen beobachtet.

## Patentansprüche

1. 5-Aminosalicylsäure-O-sulfate von physiologisch unbedenklichen Basen.

2. Verfahren zur Herstellung von 5-Aminosalicylsäure-O-sulfaten von physiologisch unbedenklichen Basen, dadurch gekennzeichnet, daß man 5-Nitrosalicylsäurealkylester mit Carbodiimiden und Schwefelsäure verestert und in den nächsten Stufen in beliebiger Reihenfolge die Alkylestergruppe alkalisch verseift und die 5-Nitrosalicylsäure-O-sulfate reduziert.

3. Verfahren gemäß Anspruch 2, dadurch gekennzeichnet, daß man als Alkylester den Ethylester einsetzt.

4. Verfahren gemäß Anspruch 2 oder 3, dadurch gekennzeichnet, daß man als Carbodiimid Dicyclohexylcarbodiimid einsetzt.

5. Verfahren gemäß Ansprüchen 2 bis 4, dadurch gekennzeichnet, daß man die alkalische Verseifung mit Kalilauge durchführt und die so erhaltenen Dikaliumsalze gewünschtenfalls nachträglich in an sich bekannter Weise in die 5-Aminosalicylsäure-O-sulfate anderer physiologisch unbedenklicher Basen überführt.

6. Arzneimittel, enthaltend 5-Aminosalicylsäure-O-sulfate von physiologisch unbedenklichen Basen.

## Claims

1. 5-Aminosalicylic acid-O-sulfates of physiologically acceptable bases.

2. A process for preparing 5-aminosalicylic acid-O-sulfates of physiologically acceptable bases, characterized in that 5-nitrosalicylic acid alkyl esters are esterified with carbodiimides and sulfuric acid and in the next steps, in optional sequence, the alkyl ester group is saponified under alkaline conditions and the 5-nitrosalicylic acid-O-sulfates are reduced.

3. The process according to claim 2, characterized in that the ethyl ester is used as the alkyl ester.

4. The process according to claims 2 or 3, characterized in that dicyclohexylcarbodiimide is used as the carbodiimide.

5. The process according to claims 2 to 4, characterized in that the alkaline saponification is carried out using aqueous potassium hydroxide and the di-potassium salts thus obtained are subsequently converted in a *per se* known manner, if desired, into the 5-aminosalicyclic acid-O-sulfates of other physiologically acceptable bases.

6. A medicament containing 5-aminosalicylic acid-O-sulfates of physiologically acceptable bases.

**Revendications**

1. O-sulfates d'acide 5-aminosalicylique de bases physiologiquement sans danger.

2. Procédé d'obtention de O-sulfates d'acide 5-aminosalicylique de bases physiologiquement sans danger, caractérisé en ce qu'on esterifie un ester alkylique d'acide 5-nitrosalicylique avec des carbodiimides et de l'acide sulfurique, et dans les étapes suivantes, dans l'ordre convenable, on saponifie à l'alcali les groupes d'esters alkyliques, et on réduit le O-sulfate d'acide 5-nitrosalicylique.

3. Procédé selon la revendication 2, caractérisé en ce qu'on utilise, comme ester alkylique, l'ester éthylique.

4. Procédé selon la revendication 2 ou 3, caractérisé en ce qu'on utilise, comme carbodiimide, la dicyclohexylcarbodiimide.

5. Procédé selon les revendications 2 à 4, caractérisé en ce qu'on opère la saponification alcaline avec une lessive potassique, et on transforme ultérieurement, si on le désire, les sels dipotassiques ainsi obtenus en les O-sulfates d'acide 5-aminosalicylique d'autres bases physiologiquement sans danger.

6. Médicament contenant des O-sulfates d'acide 5-aminosalicylique de bases sans danger.